# EUROPEAN PATENT SPECIFICATION

(11) **EP 3 437 663 B1**
(45) Date of publication and mention of the grant of the patent: **02.04.2025**
(21) Application number: 17775674.9
(22) Date of filing: 28.02.2017
(51) Int. Cl.: A61K 48/00, A61K 31/713, A61K 8/60, A23L 33/13, G01N 33/50, G01N 33/68

(54) **A NON-THERAPEUTIC USE OF A COMPOSITION FOR MOISURISING SKIN OR STRENGTHENING SKIN BARRIER FUNCTION BY PROMOTING DIFFERENTIATION OF SKIN CELLS**
NICHT-THERAPEUTISCHE VERWENDUNG EINER ZUSAMMENSETZUNG ZUR MOISURISIERUNG VON HAUT ODER ZUR VERSTÄRKUNG DER HAUTBARRIEREFUNKTION DURCH FÖRDERUNG DER DIFFERENZIERUNG VON HAUTZELLEN
UTILISATION NON THÉRAPEUTIQUE D'UNE COMPOSITION POUR MOISURISER LA PEAU OU RENFORCER LA FONCTION DE BARRIÈRE CUTANÉE EN FAVORISANT LA DIFFÉRENCIATION DES CELLULES CUTANÉESES

(30) Priority: 28.03.2016 KR 20160036834
(43) Date of publication of application: 06.02.2019
(73) Proprietor: Amorepacific Corporation, Seoul 04386 (KR)
(72) Inventor: SON, Eui Dong, Yongin-si Gyeonggi-do 17074 (KR); KIM, Hyoung June, Yongin-si Gyeonggi-do 17074 (KR); KIM, Kyu Han, Yongin-si Gyeonggi-do 17074 (KR); PARK, Sun Young, Yongin-si Gyeonggi-do 17074 (KR); LEE, Sung Hoon, Yongin-si Gyeonggi-do 17074 (KR); CHOI, Min Sik, Yongin-si Gyeonggi-do 17074 (KR); CHO, Eun-Gyung, Yongin-si Gyeonggi-do 17074 (KR); LEE, Tae Ryong, Yongin-si Gyeonggi-do 17074 (KR)
(74) Representative: Novagraaf Technologies
(86) International application number: PCT/KR2017/002211
(87) International publication number: WO 2017/171249

(56) References cited:
- WO-A1-2006/031210
- WO-A1-2014/167030
- RYUTA MUROMOTO ET AL: "Jun Activation Domain-binding Protein 1 (JAB1) Is Required for the Optimal Response to Interferons", JOURNAL OF BIOLOGICAL CHEMISTRY, vol. 288, no. 43, 25 October 2013 (2013-10-25), US, pages 30969 - 30979, XP055619799, ISSN: 0021-9258, DOI: 10.1074/jbc.M113.485847
- D'AMICO FABIO ET AL: "S100A7: A rAMPing up AMP molecule in psoriasis", CYTOKINE AND GROWTH FACTOR REVIEWS, ELSEVIER LTD, GB, vol. 32, 25 January 2016 (2016-01-25), pages 97 - 104, XP029821544, ISSN: 1359-6101, DOI: 10.1016/J.CYTOGFR.2016.01.002
- MARIAGRAZIA GRANATA ET AL: "S100A7, Jab1, and p27 kip1 expression in psoriasis and S100A7 CRISPR-activated human keratinocyte cell line", JOURNAL OF CELLULAR BIOCHEMISTRY, vol. 120, no. 3, 11 September 2018 (2018-09-11), pages 3384 - 3392, XP055620096, ISSN: 0730-2312, DOI: 10.1002/jcb.27609
- CALANDRA, THIERRY ET AL.: "Macrophage Migration Inhibitory Factor: A Regulator of Innate Immunity", NATURE REVIEWS IMMUNOLOGY, vol. 3, 2003, pages 79, XP055430236
- WEBB, MEGHAN ET AL.: "Expression Analysis of the Mouse S 100A7 /psoriasin Gene in Skin Inflammation and Mammary Tumorigenesis", BMC CANCER, vol. 5, 2005, pages 1 - 16, XP021004775
- EMBERLEY, ETHAN D. ET AL.: "The S 100A7 -c-Jun Activation Domain Binding Protein 1 Pathway Enhances Prosurvival Pathways in Breast Cancer", CANCER RESEARCH, vol. 65, no. 13, 2005, pages 5696 - 5702, XP055533222
- GESSER, B.: "Dimethylfumarate Inhibits MIF-induced Proliferation of Keratinocytes by Inhibiting MSK1 and RSK1 Activation and by Inducing Nuclear pc-Jun (S63) and p-p53 (S15) Expression", INFLAMMATION RESEARCH, vol. 60, 2011, pages 643 - 653, XP019916954

## Description

### [Technical Field]

Disclosed in the present specification is a non-therapeutic use of a composition comprising a material inhibiting the expression or activity of JAB-1 (Jun activator binding-1) for moisturizing skin or strengthening skin barrier function by promoting the differentiation of skin cells.

### [Background Art]

The epidermis is the outermost layer of the skin, and the stratum corneum is the outermost layer of the epidermis. When the stratum corneum, composed of keratinocytes, is in normal state, it can protect the skin from various external irritants and retain the moisture released from the body. The keratinocytes proliferate in the basal layer, which is the innermost skin layer, and differentiate gradually as they pass through the spinous layer and the granular layer. Through this keratinization process, the keratinocytes produce natural moisturizing factors (NMFs) and lipids (ceramides, cholesterols or fatty acids) and form the stratum corneum. The skin barrier function can work properly only when the stratum corneum is formed normally.

Skin diseases and troubles such as atopy, psoriasis or acne occur when the stratum corneum does not maintain its normal function due to various factors. They mainly exhibit skin barrier destruction and skin xerosis.
Currently, immunosuppressants, etc. are mainly used for the treatment of atopy and psoriasis, and ceramides are mainly used for strengthening the skin barrier and treating skin xerosis. However, they have disadvantages that they neither have a noticeable effect nor effect a permanent cure.

### [Summary of Invention]

### [Technical Problem]

In one aspect, an object of the present invention is to provide a non-therapeutic use of a composition comprising a material inhibiting the expression or activity of JAB-1 for moisturizing skin or strengthening skin barrier function by promoting the differentiation of skin cells.

Document Ryuta Muromoto et al.: "Jun Activation Domain-Binding Protein 1 (JAB1) Is Required for the Optimal Response to interferons" JOURNAL OF BIOLOGICAL CHEMISTRY, vol. 288, n°43, 25 October 2013, pages 30968-30979, XP055619799 discloses a nucleic acid which inhibits JAB-1 expression, and that the knockdown of Jun activation domain-binding protein 1 (JAB1) results in the reduction of IFNR level and IFN-Induced gene expression. It discloses also and antibody which specifically binds to JAB-1.

Document Ryuta Muromoto et al.: "Jun Activation Domain-Binding Protein 1 (JAB1) Is Required for the Optimal Response to interferons" JOURNAL OF BIOLOGICAL CHEMISTRY, vol. 288, n°43, 25 October 2013, pages 30968-30979, discloses a nucleic acid which inhibits JAB-1 expression, and that the knockdown of Jun activation domain-binding protein 1 (JAB1) results in the reduction of IFNR level and IFN-Induced gene expression. It discloses also and antibody which specifically binds to JAB-1.

Document WO 2006/031210 (UNIV TEXAS [US]; CLARET FRANCOIS [US], March 23, 2006, discloses JAB-1 inhibiting ligands such as antisense nucleic acids, and their formulation as pharmaceutical compositions.

Document WO 2006/031210 (UNIV TEXAS [US]; CLARET FRANCOIS [US], March 23, 2006, discloses JAB-1 inhibiting ligands such as antisense nucleic acids, and their formulation as pharmaceutical compositions.

### [Solution to Problem]

In one aspect, the present invention provides a non-therapeutic use of a composition for moisturizing skin or strengthening skin barrier function by promoting differentiation of skin cells comprising a material inhibiting the expression or activity of JAB-1 (Jun activator binding-1) as an active ingredient.

### [Advantageous Effects of Invention]

The composition for promoting differentiation of skin cells used in the present invention inhibits the expression of JAB-1 (Jun activator binding-1) or inhibits the binding between JAB-1 and psoriasin, thereby inhibiting the migration of psoriasin into the cell nucleus so as to enable the differentiation of skin cells to be effectively promoted. Therefore, the composition is effective in skin moisturization or skin barrier function strengthening.

### [Brief Description of Drawings]

FIG. 1A is a photograph showing the expression level of JAB-1 in normal human keratinocytes.
FIG. 1B is a photograph showing the expression level of JAB-1 in keratinocytes of an atopic patient (Jab 1 refers to JAB-1).
FIG. 2A is a photograph showing the expression level of S100A7 in keratinocytes not treated with interleukin-6 (IL-6).
FIG. 2B is a photograph showing the expression level of S100A7 in keratinocytes after treatment with interleukin-6 (IL-6).
FIG. 3 shows the expression level of JAB-1 in keratinocytes after treatment with interleukin-6 (left: group not treated with IL-6, right: group treated with IL-6).
FIG. 4 presents photographs showing the migration of S100A7 into the cell nucleus in the case where the cells were treated with interleukin-6 and the case where JAB-1 was knocked down (green: S100A7, red: nucleus, yellow: S100A7 which has migrated into the nucleus).
FIG. 5 is a graph showing the expression levels of keratin 1 and keratin 10 in the case where JAB-1 was knocked down.

### [Description of Embodiments]

### [Embodiments]

Hereinafter, the present invention will be described in detail.

JAB-1 (Jun activator binding-1 or Jun activation domain binding protein-1) is the first known protein that serves as a coactivator of AP-1 protein. Although JAB-1's mechanism in the proliferation of cancer cells is known, its mechanism in the differentiation of skin cells is not known yet.

The present inventors have found that psoriasin migrates into the nucleus after reaction with JAB-1 and that the expression levels of JAB-1 and psoriasin are high, particularly in atopic patients and patients with psoriasis. Based on the findings, the present inventors have found that it is possible to promote the differentiation of skin cells by inhibiting the expression of JAB-1. Specifically, the present inventors have found the following: psoriasin reacts with JAB-1 and then migrates into the cell nucleus. The psoriasin that has migrated into the cell nucleus inhibits the differentiation of skin cells. However, it is possible to promote the differentiation of skin cells by inhibiting the migration of psoriasin into the cell nucleus by inhibiting the expression of JAB-1. Psoriasin, which is a protein also called S100A7 (S100 calcium-binding protein A7), is encoded by the S100A7 gene.

In one aspect, the present invention relates to the non-therapeutic use of a composition for moisturizing skin or strengthening skin barrier function by promoting the differentiation of skin cells comprising a material inhibiting the expression or activity of JAB-1 (Jun activator binding-1) as an active ingredient.

In another aspect, the present specification relates to materials inhibiting the expression or activity of JAB-1 for moisturizing skin or strengthening skin barrier function by promoting differentiation of skin cells.

In another aspect, the present specification discloses the non-therapeutic use of materials inhibiting the expression or activity of JAB-1 as active ingredients for moisturizing skin or strengthening skin barrier function by promoting the differentiation of skin cells.

In another aspect, the present specification discloses the use of materials inhibiting the expression or activity of JAB-1 for the preparation of a composition for moisturizing skin or strengthening skin barrier function by promoting differentiation of skin.

As used herein, the expression "inhibiting the activity of JAB-1" means inhibiting the binding between JAB-1 and psoriasin.

As used herein, the term "skin" refers to tissues covering the surface of an animal body. It encompasses not only tissues covering the surface of a face or a body but also the scalp and hair. The skin as used herein includes not only the skin of a living body but also an artificial skin or a skin mimic mimicking the skin of a living body.

In one aspect of the present invention, the skin cells comprise keratinocytes. In another aspect of the present invention, the skin cells comprise human keratinocytes. In yet another aspect of the present invention, the skin cells comprise normal human keratinocytes.

As used herein, the expression "differentiation of skin cells" may mean a process in which keratinocytes functionally differentiate in the basal layer, which is the innermost skin layer, and gradually form the spinous layer, the granular layer and the stratum corneum.

As used herein, the expression "relative expression level" may mean the expression level of JAB-1 in skin cells treated with a test material compared with the expression level of JAB-1 in skin cells not treated with the test material. The expression level may include an expression amount and an expression quality.

In the above aspect, the material inhibiting the expression or activity of JAB-1 is an RNA nucleic acid molecule.

In the composition for promoting the differentiation of skin cells according to one aspect of the present invention, the RNA nucleic acid molecule is at least one of siRNA, shRNA, and miRNA which bind to the mRNA of JAB-1.

siRNA, shRNA and miRNA are RNA fragments that induce RNA interference (RNAi). RNA interference means the degradation of a target mRNA by a double stranded RNA (dsRNA), resulting in downregulation of the expression of a specific gene.

miRNA is an endogenous small RNA. It is derived from a DNA that does not synthesize proteins and generated from a hairpin-shaped transcript. The miRNA binds to the complementary sequence of the 3'-UTR of the target mRNA to induce the inhibition of the translation or the destabilization of the mRNA, ultimately serving as a repressor to inhibit protein synthesis of the target mRNA. This action is called RNAi. It is known that one miRNA can target several mRNAs, and mRNA can also be regulated by several miRNAs. Other RNAs that induce RNAi include short interfering RNA (siRNA), which is a short RNA of about 19 to 27 mer, and shRNA with a short hairpin structure. In general, siRNA and shRNA are artificially introduced into cells to induce RNAi, while miRNA is naturally present in cells.

In the above aspect, the siRNA may comprise sequences that are each capable of hybridizing with them or sequences that are each complementary to them.

As used herein, the expression "capable of hybridizing" means capable of binding to a specific single stranded RNA to form a double stranded RNA.

The composition for promoting the differentiation of skin cells according to one aspect of the present invention may inhibit the migration of psoriasin into the cell nucleus. Specifically, psoriasin binds to JAB-1 and then migrates into the cell nucleus to inhibit cell differentiation, but the composition can inhibit this mechanism.

In one embodiment, the skin cells may be keratinocytes.

In the above aspect, the composition is a composition for moisturizing the skin or strengthening the skin barrier function by promoting differentiation of skin cells. If the differentiation of skin cells is not performed properly, the stratum corneum cannot perform its normal function, which leads to reduction of the water holding capacity of the skin and deterioration of the skin barrier function. Further, skin diseases such as atopy and psoriasis occur when the stratum corneum does not maintain its normal function due to various factors. The major symptoms of the diseases are skin inflammation and skin xerosis. Thus, materials promoting the differentiation of skin
cells can exert the effects of skin moisturization, and skin barrier function strengthening.

The composition for promoting the differentiation of skin cells is a cosmetic composition, or a health food composition.

The cosmetic composition may be provided in any formulation suitable for topical application, for example, a solution, an oil-in-water emulsion, a water-in-oil emulsion, a suspension, a solid, a gel, a powder, a paste, a foam, or an aerosol composition. These formulations may be prepared according to methods commonly employed in the art.

The cosmetic composition may comprise other ingredients which do not negatively affect the main effect of the present invention, and which preferably provide a synergic effect to the main effect. The cosmetic composition of the present invention may comprise a material selected from the group consisting of vitamins, polypeptides, polysaccharides, and sphingolipids. Also, the cosmetic composition of the present invention may comprise a humectant, an emollient, a surfactant, a UV absorbent, a preservative, a sterilizer, an antioxidant, a pH adjuster, an organic or inorganic pigment, a fragrance, a cooling agent, an antiperspirant or the like. The amount of these ingredients may be easily determined by those skilled in the art within a range not negatively affecting the purpose and effect of the present invention. The content of the ingredients may be 0.01-5% by weight, specifically, 0.01-3% by weight, based on the total weight of the composition.

The health food may refer to a food prepared with raw materials or ingredients (functional raw materials) having a nutrient likely to be deficient in normal diets or a function useful for the human body, and which helps to maintain and improve health by maintaining the normal function or activating the physiological function of the human body, although not limited thereto. The health food may be prepared and processed in the form of a tablet, a capsule, a powder, a granule, a liquid, a pill or the like. However, the health food is not limited thereto and may be prepared and processed in any form according to the law.

The health beverage composition according to one aspect of the present invention has no particular limitation on other ingredients, except that a predetermined amount of the above compound is included as an essential ingredient. It may comprise various flavoring agents, natural carbohydrates or the like as additional ingredients, as do common beverages. Examples of the natural carbohydrates include conventional sugars such as monosaccharides, polysaccharides, and cyclodextrins and sugar alcohols such as xylitol, sorbitol, and erythritol. Also, natural flavoring agents (thaumatin, stevia extract (for example, rebaudioside A, glycyrrhizin, etc.)) and synthetic flavoring agents (for example, saccharin, aspartame, etc.) may be used as the flavoring agents.

In general, the daily dose of the active ingredient of the health food composition may be 0.0001 mg/kg/day to about 1000 mg/kg/day, preferably 0.02mg/kg/day to 100mg/kg/day. It may be administered once a day or in several divided doses per day.

In the composition for promoting differentiation of skin cells according to one aspect of the present invention, the material inhibiting the expression or activity of JAB-1 may be included at a concentration of 0.1 nM to 100 nM.

In addition, the content of the material inhibiting the expression or activity of JAB-1 may be 0.0001 to 30% by weight, for example, 0.0001 to 25% by weight, 0.0001 to 25% by weight, 0.0001 to 20% by weight or 0.0001 to 15% by weight, preferably 0.0001 to 10% by weight, based on the total weight of the composition.

The present specification relates to a method for screening for materials promoting the differentiation of skin cells, comprising the steps of: treating skin cells with test materials; and identifying the relative expression levels of JAB-1 in the skin cells before and after treatment with the test materials.

The screening method may further comprise the step of, if the expression level of JAB-1 after treatment with test materials is lower than the level before the treatment, determining that the test materials are materials promoting the differentiation of skin cells. In one embodiment, the screening method may further comprise the step of, if the expression level of JAB-1 after treatment with test materials is at least 20% lower than the level before the treatment, determining that the test materials are materials promoting the differentiation of skin cells. For example, if the expression level of JAB-1 after treatment with test materials is at least 15%, at least 20%, at least 25%, at least 30%, at least 35%, at least 40% or at least 50% lower than the level before the treatment, the test materials can be determined to be materials promoting the differentiation of skin cells.

For example, if the expression level of JAB-1 in keratinocytes treated with test materials is higher than the expression level of JAB-1 in keratinocytes not treated with the test materials, it can be determined that the test materials increased the expression level of JAB-1. On the contrary, if the expression level of JAB-1 in skin cells treated with test materials is lower than or similar to the expression level of JAB-1 in skin cells not treated with the test materials, it can be determined that the test materials did not increase the expression level of JAB-1. As described above, if test materials reduce the expression level of JAB-1 by at least 20% compared to the level before the treatment, the test materials can be determined to be materials promoting the differentiation of skin cells.

The expression level of JAB-1 may be identified by a known technology, for example, reverse transcription-polymerase chain reaction (RT-PCR), ELISA, western blot or immune blot, although not limited thereto.

It can also be visually identified whether or not the expression level of JAB-1 has decreased.

The screening method may further comprise the step of identifying the relative expression level of at least one of keratin 1 and keratin 10 before and after treatment with the test materials. The method may further comprise the step of, if the expression level of at least one of keratin 1 and keratin 10 after treatment with test materials is higher than the level before the treatment, determining that the test materials are materials promoting the differentiation of skin cells. In one embodiment, the screening method may further comprise the step of, if the expression level of at least one of keratin 1 and keratin 10 after treatment with test materials is at least 20% higher than the level before the treatment, determining that the test materials are materials promoting the differentiation of skin cells. For example, if the expression level of keratin 1 or keratin 10 after treatment with test materials is at least 15%, at least 20%, at least 25%, at least 30%, at least 35%, at least 40%, at least 45% or at least 50% higher than the level before the treatment, the test materials can be determined to be materials promoting the differentiation of skin cells.

In another aspect, the present specification disclosed that keratin 1 and keratin 10 can serve as markers of differentiation of skin cells. If keratin 1 and keratin 10 are highly expressed, it can be determined that skin cell differentiation is active.

In one embodiment, the skin cells may be keratinocytes, although not limited thereto.

### [Examples]

Hereinafter, the constitution and effects of the present invention will be described in more detail through examples. However, the following examples are provided for illustrative purposes only to facilitate understanding of the present invention, and the scope of the present invention is not limited thereto.

### Example 1: Measurement of the expression levels of JAB-1 in keratinocytes of a normal human and an atopic patient

The expression levels of JAB-1 in the skin tissues of an atopic patient and a normal human were identified. The skin tissues of an atopic patient were obtained from the dermatology department of Chung-Ang University.

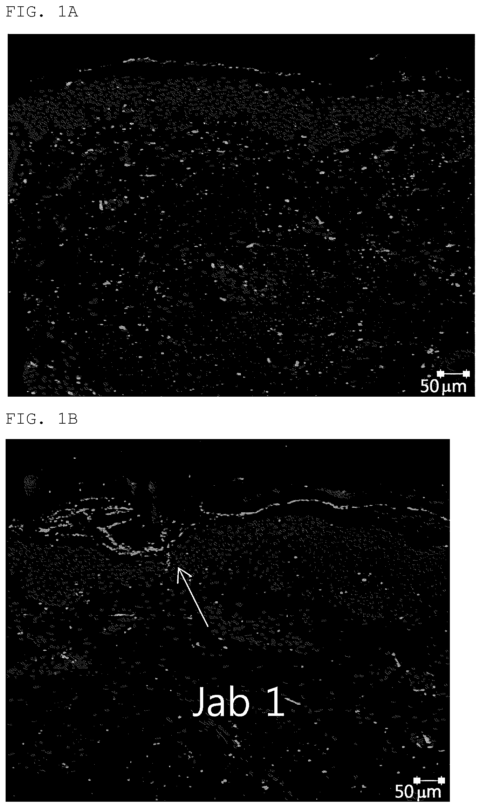

2-mm epidermis biopsy samples were obtained from the normal group (age 32, male) and the atopy group (age 24, male). The samples were then embedded in a frozen section compound (FSC 22^{®}, Leica Microsystems, USA) and cut into 6 µm sections. The tissue slides were immunostained using a reagent for cell nuclear staining, DAPI (4',6-diamidino-2-phenylindole) and an anti-JAB-1 antibody. From the results, it was seen that in the normal group, blue (less bright portion) stained nuclei were found throughout the epidermal layer while no stained JAB-1 (green; brighter portion) was observed (FIG. 1A). In contrast, in the atopic tissues, green (brighter portion) stained JAB-1 increased in the upper part of the epidermis and stained nuclei were found (FIG. 1B). Thus, it can be understood that the expression level of JAB-1 was higher in the skin
(epidermis) of the atopic patient.

### Example 2: Measurement of the expression levels of S100A7 and JAB-1 when treated with in the case of application of interleukin-6 (IL-6)

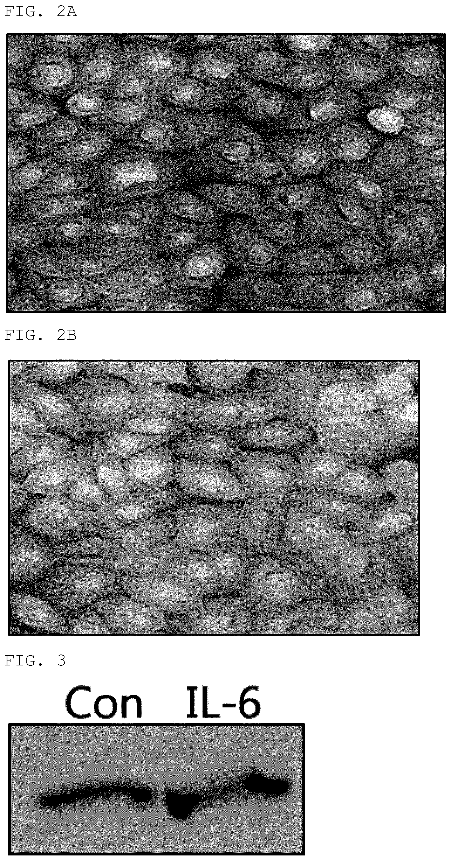

Keratinocytes were purchased from Lonza. Each cell was cultured in a CO₂ incubator under the conditions of 37°C and 5% CO₂. The cell culture was performed according to Lonza's guidelines. The medium used in the cell culture was prepared by adding 2 ml of a KGM-2 bullet kit (bovine pituitary extract (BPE)), 0.5 ml of human epidermal growth factor (hEGF), 0.5 ml of insulin, 0.5 ml of hydrocortisone, 0.5 ml of transferrin, 0.5 ml of epinephrine, and 0.5 ml of gentamycin sulfate and amphotericin-B (GA-1000) to 500 ml of KGM-2 (Clonetics CC-3103) medium. The keratinocytes were cultured in a 60 mm culture dish to a confluency of 70-80%. Then, in order to investigate the expression level of S100A7, which is a protein binding to JAB-1, and the expression level of JAB-1 when treated with an inflammatory cytokine IL-6 (10 nM/ml), immunostaining was performed using an anti-S100A7 antibody to determine the expression level of S100A7 (FIG. 2A and FIG. 2B) and western blot was performed to determine the expression level of JAB-1 (FIG. 3).

From the results, it was found that the group treated with IL-6 showed higher expression levels of S100A7 and JAB-1, stained in green (slightly bright portions in the cells), than the group not treated with IL-6 (FIG. 2A, FIG. 2B and FIG. 3). Interestingly, it can be seen that a large amount of S100A7 is found in the nuclei of the group with increased expression of JAB-1. The yellow portions (very bright portions inside the circles) in FIG. 2A and FIG. 2B resulted from increased nuclear translocation of S100A7.

### Example 3: Evaluation of the migration of S100A7 into the cell nucleus after knockdown of JAB-1

Cells were treated with IL-6 according to the same method as Example 2 and transfected with siRNA of the JAB-1 gene (50 nM, ON-TARGET plus siRNA Reagents purchased from Dharmacon) using lipofectamine to knock down JAB-1. Then, it was observed whether S100A7 migrated into the nucleus.

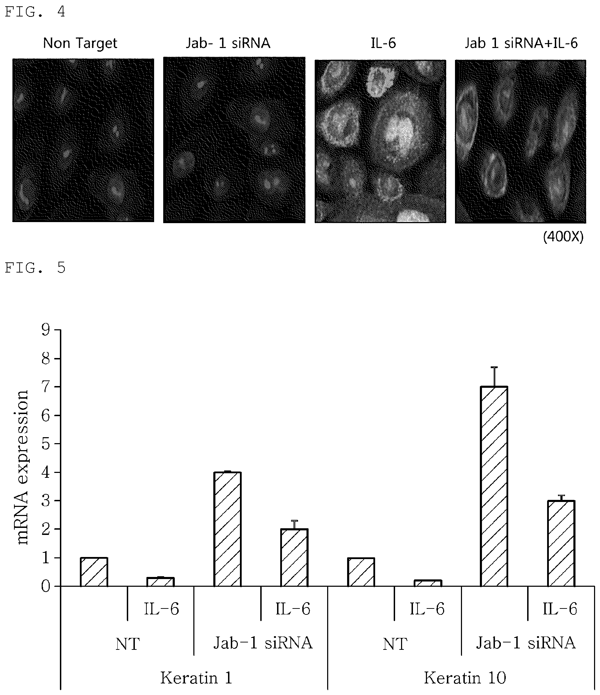

Propidium iodide was used for nuclear staining, and a fluorescence labeled anti-S100A7 antibody (Abcam ab13680) was used for staining S100A7. The stained nuclei were red and the stained S100A7 was green. If S100A7 migrates into the nucleus, red and green are combined to give yellow. From the results, it was found that in the group treated with IL-6, green S100A7 was present as yellow portions in the nuclei (the brightest portions in the third figure in FIG. 4), but in the case where JAB-1 was knocked down, yellow was hardly observed, indicating that almost no S100A7 migrated into the nucleus (FIG. 4).

### Example 4: Measurement of the mRNA expression level of keratin 1 and keratin 10 after knockdown of JAB-1

After treatment with IL-6 and JAB-1 knockdown according to the same method as Example 3, the mRNA expression levels of keratin 1 and keratin 10, which are epidermal differentiation markers, were measured. From the results, it was found that the expression levels of mRNAs of keratin 1 and keratin 10 increased when JAB-1 was knocked down, and that the epidermal differentiation markers expression reduced by IL-6 was restored when JAB-1 was knocked down (FIG. 5).

### Example 5: Measurement of the mRNA expression level of JAB-1 after knockdown of JAB-1

Two days after knocking down JAB-1 according to the same method as Example 3, the cells were cultured, the medium was removed, 1 ml of Trizol (Invitrogen) was added and RNA was isolated according to the RNA isolation method of Invitrogen for comparison with the control group (non-targeting RNA, 50 nM). RNA was quantified using an ultraviolet detector (Hewlett Packard) at 260 nm and subjected to reverse transcription-polymerase chain reaction (RT-PCR). For gene analysis of each sample, normalization was performed with the complementary gene RPL13A.

From the results, it was found that in the JAB-1 knockdown group, the gene expression level of JAB-1 decreased by about 20-70% in comparison with the control group. The test results are shown in Table 1.

**Table 1**

| | Control group (non-targeting RNA, 50 nM) | Test group (JAB-1 siRNA, 50 nM) |
|---|---|---|
| Comparison of the expression level of JAB-1 gene | 100% | 20-70% |

Hereinafter, formulation examples of the composition according to one aspect of the present invention will be described. However, it may be formulated into various other forms. These examples are for illustrative purposes only and are not intended to limit the scope of the present invention.

### Formulation Example 1: Milk lotion

A milk lotion can be prepared according to a conventional method with the composition shown in Table 2 below.

**Table 2**

| Ingredient | Content (% by weight) |
|---|---|
| Glycerin | 3.0 |
| Butylene glycol | 3.0 |
| Propylene glycol | 3.0 |
| Carboxyvinyl polymer | 0.1 |
| 50 nM siRNA of JAB-1 of Example 3 | 0.01 |
| Wax | 4.0 |
| Polysorbate 80 | 1.5 |
| Caprylic/capric triglyceride | 5.0 |
| Squalane | 5.0 |
| Sorbitan sesquioleate | 1.5 |
| Cetearyl alcohol | 1.0 |
| Triethanolamine | 0.2 |
| Preservative, fragrance | q. s. |
| Purified water | Balance |
| Total | 100 |

### Formulation Example 2: Nourishing lotion

A nourishing lotion can be prepared according to a conventional method with the composition shown in Table 3 below.

**Table 3**

| Ingredient | Content (% by weight) |
|---|---|
| Purified water | Balance |
| Glycerin | 3.0 |
| Butylene glycol | 3.0 |
| Liquid paraffin | 5.0 |
| Beta-glucan | 7.0 |
| Carbomer | 0.1 |
| 50 nM siRNA of JAB-1 of | 0.01 |
| Example 3 | |
| Caprylic/capric triglyceride | 3.0 |
| Squalane | 5.0 |
| Cetearyl glucoside | 1.5 |
| Sorbitan stearate | 0.4 |
| Polysorbate 60 | 1.5 |
| Preservative | q. s. |
| Fragrance | q. s. |
| Pigment | q. s. |
| Triethanolamine | 0.1 |
| Total | 100 |

### Formulation Example 3: Nourishing cream

A nourishing cream can be prepared according to a conventional method with the composition shown in Table 4 below.

**Table 4**

| Ingredient | Content (% by weight) |
|---|---|
| Glycerin | 3.5 |
| Butylene glycol | 3.0 |
| Liquid paraffin | 7.0 |
| Beta-glucan | 7.0 |
| Carbomer | 0.1 |
| 50 nM siRNA of JAB-1 of Example 3 | 0.01 |
| Caprylic/capric triglyceride | 3.0 |
| Squalane | 5.0 |
| Cetearyl glucoside | 1.5 |
| Sorbitan stearate | 0.4 |
| Polysorbate 60 | 1.2 |
| Triethanolamine | 0.1 |
| Preservative, fragrance | q. s. |
| Purified water | Balance |
| Total | 100 |

### Formulation Example 4: Pack

A pack can be prepared according to a conventional method with the composition shown in Table 5 below.

**Table 5**

| Ingredient | Content (% by weight) |
|---|---|
| Glycerin | 4.0 |
| Polyvinyl alcohol | 15.0 |
| Hyaluronic acid extracts | 5.0 |
| Beta-glucan | 7.0 |
| Allantoin | 0.1 |
| 50 nM siRNA of JAB-1 of Example 3 | 0.01 |
| Nonyl phenyl ether | 0.4 |
| Polysorbate 60 | 1.2 |
| Ethanol preservative | q. s. |
| Preservative, fragrance | q. s. |
| Purified water | Balance |
| Total | 100 |

### Formulation Example 5: Ointment

An ointment can be prepared according to a conventional method with the composition shown in Table 6 below.

**Table 6**

| Ingredient | Content (% by weight) |
|---|---|
| Glycerin | 8.0 |
| Butylene glycol | 4.0 |
| Liquid paraffin | 15.0 |
| Beta-glucan | 7.0 |
| Carbomer | 0.1 |
| 50 nM siRNA of JAB-1 of Example 3 | 0.01 |
| Caprylic/capric triglyceride | 3.0 |
| Squalane | 1.0 |
| Cetearyl glucoside | 1.5 |
| Sorbitan stearate | 0.4 |
| Cetearyl alcohol | 1.0 |
| Preservative | q. s. |
| Fragrance | q. s. |
| Pigment | q. s. |
| Wax | 4.0 |
| Purified water | Balance |
| Total | 100 |

### Formulation Example 6: Injection

An injection can be prepared according to a conventional method with the composition shown in Table 7 below.

**Table7**

| Ingredient | Content |
|---|---|
| 50 nM siRNA of JAB-1 of Example 3 | 10-50 mg |
| Ovalbumin | 0.01-0.5 mg |
| Sterilized distilled water for injection | q. s. |
| pH adjuster | q. s. |

## Claims

1. A non-therapeutic use of a composition comprising a material inhibiting the expression or activity of JAB-1 (Jun activator binding-1) for moisturizing skin or strengthening skin barrier function by promoting differentiation of skin cells,
wherein the material inhibiting the expression or activity of JAB-1 is an RNA nucleic acid molecule which is at least one of siRNA, shRNA, and miRNA which bind to the mRNA of JAB-1, and
wherein the material inhibiting the expression or activity of JAB-1 is formulated in a form of a composition which is a cosmetic composition or a health food composition.

2. The non-therapeutic use of a composition according to claim 1,
wherein the material inhibiting the expression or activity of JAB-1 (Jun activator binding-1) inhibits the migration of psoriasin into the cell nucleus.

3. The non-therapeutic use of a composition according to claim 1 or 2, wherein the skin cells are keratinocytes.

4. The non-therapeutic use of a composition according to any one of claims 1 to 3,
wherein the composition comprises the material inhibiting the expression or activity of JAB-1 at a concentration of 0.1 nM to 100 nM, or
wherein the content of the material inhibiting the expression or activity of JAB-1 is 0.0001 to 30% by weight based on the total weight of the composition.

## Patentansprüche

1. Nicht-therapeutische Verwendung einer Zusammensetzung, die ein Material umfasst, das die Expression oder Aktivität von JAB-1 (Jun activator binding-1) hemmt, zur Befeuchtung der Haut oder zur Stärkung der Hautbarrierefunktion durch Förderung der Differenzierung von Hautzellen,
wobei das Material, das die Expression oder Aktivität von JAB-1 hemmt, ein RNA-Nukleinsäuremolekül ist, bei dem es sich um mindestens eine von siRNA, shRNA und miRNA handelt, die an die mRNA von JAB-1 binden, und
wobei das Material, das die Expression oder Aktivität von JAB-1 hemmt, in Form einer Zusammensetzung formuliert ist, die eine kosmetische Zusammensetzung oder eine Gesundheitsnahrungszusammensetzung ist.

2. Die nicht-therapeutische Verwendung einer Zusammensetzung nach Anspruch 1,
wobei das Material, das die Expression oder Aktivität von JAB-1 (Jun activator binding-1) hemmt, die Migration von Psoriasin in den Zellkern hemmt.

3. Nichttherapeutische Verwendung einer Zusammensetzung nach Anspruch 1 oder 2, wobei die Hautzellen Keratinozyten sind.

4. Die nicht-therapeutische Verwendung einer Zusammensetzung nach einem der Ansprüche 1 bis 3,
wobei die Zusammensetzung das Material umfasst, das die Expression oder Aktivität von JAB-1 in einer Konzentration von 0,1 nM bis 100 nM hemmt, oder
wobei der Gehalt an dem Material, das die Expression oder Aktivität von JAB-1 hemmt, 0,0001 bis 30 Gew.-%, bezogen auf das Gesamtgewicht der Zusammensetzung, beträgt.

## Revendications

1. Utilisation non thérapeutique d'une composition comprenant un matériau inhibant l'expression ou l'activité de JAB-1 (Jun activator binding-1) pour hydrater la peau ou renforcer la fonction de barrière cutanée en favorisant la différenciation des cellules de la peau,
dans lequel le matériel inhibant l'expression ou l'activité de JAB-1 est une molécule d'acide nucléique ARN qui est au moins l'un des siRNA, shRNA et miRNA qui se lient à l'ARNm de JAB-1, et
dans lequel le matériau inhibant l'expression ou l'activité de JAB-1 est formulé sous la forme d'une composition qui est une composition cosmétique ou une composition d'aliment de santé.

2. Utilisation non thérapeutique d'une composition selon la revendication 1,
dans lequel le matériau inhibant l'expression ou l'activité de JAB-1 (Jun activator binding-1) inhibe la migration de la psoriasine dans le noyau cellulaire.

3. Utilisation non thérapeutique d'une composition selon la revendication 1 ou 2, dans laquelle les cellules de la peau sont des kératinocytes.

4. Utilisation non thérapeutique d'une composition selon l'une des revendications 1 à 3,
dans laquelle la composition comprend la substance inhibant l'expression ou l'activité de JAB-1 à une concentration de 0,1 nM à 100 nM, ou
dans laquelle la teneur en matière inhibant l'expression ou l'activité du JAB-1 est comprise entre 0,0001 et 30 % en poids par rapport au poids total de la composition.
